(19) Europäisches Patentamt
European Patent Office
Office européen des brevets

(11) **EP 4 480 963 A1**

(12) **EUROPEAN PATENT APPLICATION**

(43) Date of publication:
**25.12.2024  Bulletin 2024/52**

(21) Application number: **23180633.2**

(22) Date of filing: **21.06.2023**

(51) International Patent Classification (IPC):
**C07K 14/72** (2006.01)       **C07K 14/59** (2006.01)
**G01N 33/76** (2006.01)       **C12Q 1/6897** (2018.01)

(52) Cooperative Patent Classification (CPC):
**C12Q 1/6897; C07K 14/59; C07K 14/723;
G01N 33/76**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **Ares Trading S.A.
1170 Aubonne, VD (CH)**

(72) Inventors:
• **RICCI, Marina
10010 Colleretto Giacosa TO (IT)**

• **NEVELLI, Francesco
10010 Colleretto Giacosa TO (IT)**
• **DADONE, Aurora
10010 Colleretto Giacosa TO (IT)**
• **VIGNA, Virginia
10010 Colleretto Giacosa TO (IT)**

(74) Representative: **Merck Serono S.A.
Intellectual Property
Terre Bonne Business Park
Building Z0
Route de Crassier 1
1262 Eysins (CH)**

(54) **CELL LINE FOR EXPRESSION OF FSH RECEPTOR**

(57)    The invention described herein relates to novel cell lines for the expression of a human FSH receptor together with a luciferase reporter gene.

Also described are methods of preparing such cell lines. The cell line of the present invention expressing the FSH receptor, and the luciferase reporter gene is useable in a method for determining the presence and the biological activity of r-FSH samples.

EP 4 480 963 A1

**Description**

*Field of the Invention:*

Described herein are novel cell line useful in the development of bioassays relevant in the manufacturing of recombinant human Follicle Stimulating Hormone (r-hFSH). The cell line expresses both the human FSH receptor and a luciferase reporter gene under the control of a cAMP response element (CRE) (HEK 293T FSHR Luciferase).

*Background of the Invention:*

Bioassays are used to evaluate the drug potency before commercial products release on the market. To estimate drug potency, the assays should reflect or mimic the product's known mechanism of action (or part of it) that occurs in the human body, to reliably evaluate the product biological activity.

In vivo potency assays are methods where animals are treated with Standard and Test materials and the relationship between product concentration and obtained response is evaluated to estimate the potency. Instead, cell-based (or in vitro) potency assays foresee the use of cell lines able to respond to specific ligands or infectious agents. The nature of cell lines can be different: they can be derived from tumors, immortalized as factor-dependent, or engineered cell lines transfected with appropriate target of interest. One of the main requirements is that these cell lines must be stable to guarantee assays reproducibility over time and reliable results delivery. Cellular responses to the protein of interest are related to the drug's mechanism of action and the duration of exposure. Some drug responses include cell proliferation, cell killing, cell differentiation, cytokine or other molecule secretion, intra-cellular response activation and enzyme activation.

Currently, the bioassays to determine presence and potency for recombinant human FSH involve an in vivo assay including the use of animals and a newly developed in vitro one. This cell-based assay foresees the use of a cell line used to determine the presence and potency of r-hFSH.

*Summary of the Invention:*

The current invention is providing a solution to the dependency to a commercial kit, which is currently used in the cell-based assay, for the execution of the test. The solution provides a new mammalian cell line that has been made to highly express the human FSH receptor and which is coupled to a reporter. As such, the binding of r-FSH to the human FSH receptor on the new cell of the present invention allows the quantification of r-FSH biological activity.

In one embodiment is provided a mammalian cell line, which already expresses a luciferase reporter gene under the control of a cAMP Response Element (CRE), for expressing a human FSH receptor comprising a nucleic acid molecule encoding the human FSH receptor under the control of a promoter for protein expression.

In another embodiment is provided a method of producing a human FSH receptor in a mammalian cell comprising: a) transfecting a mammalian cell with a lentivirus containing the gene encoding the FSH receptor under the control of a promoter for protein expression, b) adding a first selectivity agent to the transfected cells of step a), to induce stably expressing human FSH receptor cells and the luciferase reporter, and d) harvesting the cells stably expressing the human FSH receptor and the luciferase reporter (HEK 293-T FSHR Luciferase).

In yet another embodiment there is provided a method for biological activity quantification of r-hFSH samples, comprising: a) stimulating the mammalian cell expressing the human FSH receptor and a luciferase reporter with the samples, b) adding luminescence substrate for the luciferase enzyme to the cell culture, c) detecting the luminescence of the cells, d) quantifying the amount of luminescence, and e) determining the potency of r-hFSH.

*Figures*

Figure 1A and 1B: FRET analysis for FSH receptor activity quantification for evaluation of transfection efficiency. HEK 293-T FSHR Luciferase (1A) vs HEK 293-T WT (1B) responses to stimulation with r-hFSH.

Figure 2: Representative RGA dose-response curves for potency quantification of r-hFSH samples

Figure 3: FSHR staining on HEK 293-T FSHR luciferase. Data were acquired using BD FACS Aria Fusion and analyzed using Flowjo software. FACS staining with anti-human FSH R Alexa Fluor® 488-conjugated Antibody of HEK 293-T WT (left panel) and HEK 293-T FSHR Luciferase (right panel).

Figure 4: Monitoring of cell transfection stability during a defined range of passages. FACS staining with anti-human FSH R Alexa Fluor®488-conjugated Antibody of HEK 293-T LHCGR Luciferase (right panel) monitored over time

Figure 5: Cellular behavior during a defined range of passages. A) Analysis of cellular viability, B) Analysis of cellular doubling. Fig 5A. Cell line viability, expressed as a percentage, performed using Nucleocounter instrument. Fig 5B. Results of the doubling time of HEK 293-T FSHR Luciferase cells show a range between 16 and 20 hours.

## Detailed Description of the Invention:

Currently, potency test for each new FSH Drug Product batch release is performed with an *in vivo* or an in vitro method. The *in vivo* method, which used rats for quality control purpose, should be performed as required and described by the regulatory authorities and guidelines. The cell-based assay one is performed for the Drug Substance batch release in a list of countries where the switch to the new method was approved,

Currently the only cell line available on the market that can be suitable for the development of an in-vitro cell based analytical method, foresees the quantification of cAMP produced by the cells after stimulation with r-hFSH. The quantification is based on a commercial kit measures the cAMP released by the cells. This technology constrains the QC staff to the use of an expensive and difficult to replace kit while it would be preferrable to use a quantification reagent with available alternatives on the market.

The present invention provides such cell line as well as a method of producing such cell line and a method for *in vitro* testing r-hFSH from a production batch using the new cell line.

The cell line of the present invention is based on a human embryonic kidney (HEK) cell line, more specifically a HEK 293-T.

The development of cell line and *in vitro* method were performed according to r-hFSH mechanism of action to comply with guidelines and health authorities' requirements.

Cell line development started from HEK 293-T Luciferase cells. The cell line HEK 293-T Luciferase was stably transfected with the human FSH receptor.

The newly created cells will faithfully reproduce a part of the *in vivo* r-hFSH mechanism of action: the first steps of the metabolic cascade produced by the receptor binding with the hormone.

The transfection processes were resulting in a new cell line of HEK 293 T cells expressing a human FSH receptor and a luciferase reporter (HEK 293-T FSHR Luciferase). The transfected cells were selected and expanded with consequent freezing of different cell banks. After cell banks creation, two different functional assays were developed with the aim to obtain a method able to preliminary assess the transfections efficiency under a functional point of view.

The first functional assay was developed for quantifying the cAMP, using FRET commercial kit, after cells stimulation. In this way, the assay allowed the assessment of FSH receptor activation in HEK 293-T FSHR Luciferase cells. Thanks to this assay it has been possible to appreciate the differences between transfected and non-transfected cells, confirming the expression of the FSH receptor only in transfected cells.

The second functional assay was developed to assess the reporter gene transcription after intracellular cAMP accumulation. This assay was carried out stimulating the cells with r-hFSH with consequent adenylate cyclase activation. Thanks to this stimulation, intracellular cAMP increased, and the reporter gene transcription should be evaluated in transfected cells. Also in this case, it has been possible to observe the differences in luminescence production, after cells stimulation, between transfected and non-transfected cells. Thanks to this assay it has been possible to confirm the presence and the functionality of the luciferase reporter gene vector in HEK 293-T FSHR Luciferase cell line.

A preliminary characterization study on HEK 293-T FSHR Luciferase was performed during a defined range of passages, from passage 20 until passage 37. During this study, cells behavior was monitored assessing specific parameters like FSH receptor expression in cells membrane, cells viability, cells proliferation (cells doubling time). Also, the previously developed functional assay was included in the characterization study, allowing the monitoring of transfection stability over time.

The FSH receptor expression in HEK 293-T FSHR Luciferase membrane was assessed, in comparison with non-transfected cells. Thanks to these experiments it has been possible to confirm the presence of the FSH receptor in transfected cells, obtaining a percentage of FSH positive cells around 95-99%.

The phenotypic and functional characterization of HEK 293-T FSHR Luciferase cell line demonstrated that cells presented stable viability and stable proliferation during the selected range of passages.

The receptor expression stability in cells membrane was evaluated by FACS analysis and stimulating the cells with r-hFSH, thus confirming the transfection stability and functionality over time.

Beside all the activities related to the cell line development and characterization, the *in vitro* assay for r-hFSH sample potency quantification was developed. The developed method procedure resulted very quick and easy; these characteristics ensure an optimal application of the assay in a quality control routine.

Examples:

Culturing of HEK 293 T Luciferase cells. The recommended cell culture medium is Dulbecco's Modified Eagle's Medium (DMEM) with the addition of 10% Fetal Bovine Serum (FBS) and 0.5 μg/mL puromycin. Cells doubling time is about 16-20 hours. Cells were cultured at $37 \pm 1°C$ in a humidified atmosphere with $5 \pm 1\%$ CO2. All these cell characteristics are described in cells datasheet provided by the supplier while the cell subculturing conditions have been identified in our laboratory.

Transfection of HEK 293T Luciferase cells. The HEK 293T Luciferase cell line was transfected with the FSH receptor using an appropriate lentivirus (provided by Vector Builder) comprising a nucleic acid molecule encoding the human FSH receptor. The vector, a mammalian gene expression vector, was 8085 bp. For a stable transfection, hygromycin B was used as a selection marker. Before transfection it was identified the minimum hygromycin B concentration able to interrupt HEK 293-T growth in 10 days performing a killing curve assay. The concentration of 200 μg/mL was identified as the best.

The lentivirus containing the gene for FSH receptor, was initially introduced into HEK 293-T Luciferase cells using cell transduction. The commercial reagents used for the transduction were included in the VectorBuilder kit, sold together with the lentivirus.

The medium used during the transduction was the OptiMEM (Life Technologies, 31985). Different MOI of infection were tested, using different amounts of vector DNA. The transduction process, as well as the different experimental conditions, were performed according to the protocol suggested by the supplier. 24 hours after transduction, the medium was replaced to remove the lentivirus with DMEM and 10% FBS. 24 hours after the medium change, the antibiotic selection was added to the cells with the aim to select only the population able to resist to the antibiotic concentration, identified during the kill curve experiment.

Resistant cells were expanded and different cell banks belonging to each transfection condition were frozen. The usual approach for the creation of a cell bank foresees the freezing of a Master Cell Bank (MCB). If needed a Working Cell Bank (WCB) will be created, starting from the MCB. Banks creation has the aim to ensure the availability of a sufficient number of cells during the development and the execution of an assay. The bank generation depends on cells growth characteristics, while the size of a bank depends on the number of cells required for each assay and how often the assay will be performed.

First functional assay evaluation. In order to evaluate the transfection efficiency, transfected cells were stimulated with r-hFSH and cAMP production was quantified using a commercial kit based on Fluorescence Resonance Energy Transfer (FRET). The FRET commercial kit used to quantify cAMP was provided by Cisbio. The kit includes a cAMP coupled with d2 (acceptor) and an antibody anti-cAMP conjugated with a cryptate (donor, Europium). The FRET is detected by a plate reader equipped with the Homogeneous Time Resolve Fluorescence (HTRF) detector technology. All the experiments were performed according to the datasheet provided by the kit supplier.

For data interpretation, the ratio between acceptor and donor emission signals must be calculated for each well using the following formula:

$$\text{Ratio} = (\text{Signal 665 nm}) / (\text{Signal 620 nm}) * 10000.$$

All the raw data (Ratio) produced with the FRET kit, were analyzed, and graphed with the statistical software GraphPad Prism.

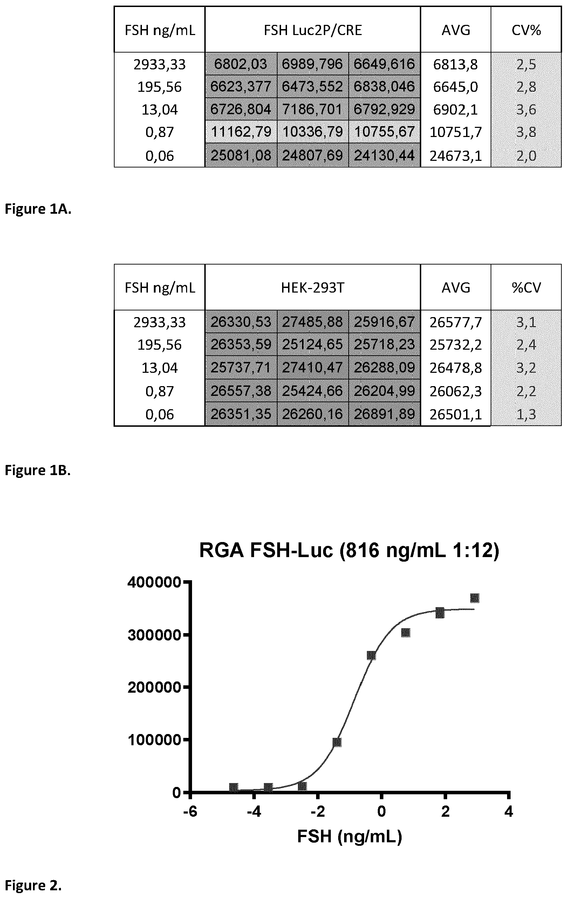

Cells were plated in a 96-well plate at the same concentration and stimulated with different amount of r-hFSH, creating a dose-response curve. As negative control, HEK 293-T without transfection were also stimulated. After 30 minutes of incubation, FRET reagents were added in each well of the plate. The plate was again incubated under shaking conditions for 60 minutes and finally the results were acquired with a plate reader. In Figure 1 panel A and B it is possible to observe how the responses produced by the two cell lines were different. Transfected cells (Figure 1A) respond to r-hFSH stimulation whereas non-transfected HEK 293-T (Figure 1B) were not able to produce a dose-related response after stimulation, confirming the absence of the receptor in cells membrane.

Second functional assay evaluation. In order to assess the transfection efficiency, a Reporter Gene Assay (RGA) was performed. This assay was suggested in the Promega vector datasheet. HEK 293-T FSHR Luciferase cells were stimulated with r-hFSH. The luminescence emitted after Reporter Gene Transcription, was quantified using reagents available on the market.

HEK 293-T FSHR Luciferase cells were seeded in a 96-well plate and stimulated with different concentration of r-hFSH. After incubation, the ONE-Glo EX Luciferase assay system (Promega, E8120) was added in each well and, after 10 minutes shaking, results were acquired using the plate reader.

In Figure 2 is shown the luminescence response (expressed as Relative Lights Units, RLUs) obtained for each well after plate reading. HEK 293-T FSHR Luciferase responses demonstrated to be dose-related. These data confirmed the success of the transfection.

<u>Cell line characterization</u>. The aim of the study was to monitor and evaluate different parameters, to investigate cell line behavior over time. Cell line behavior evaluation is important to monitor cells viability, proliferation rate, target receptor expression and functional performances for a determine number of cell passages. The monitored parameters for HEK 293-T FSH Luciferase cells were:

- Presence of FSH receptor expression in cells membrane and its stability over time,
- Cells viability,
- Cells proliferation (doubling time).

Cells were monitored for a long-time starting from passage 20 until passage 37.

Each parameter was assessed during the defined range of passages. Data were analyzed using linear regression analysis at the 95% of regression significance (p-value <0.05). All statistical analyses were performed using Graph Pad Prism.

<u>FSH receptor expression assessment in cells membrane and its stability over time</u>. The percentage of transfected cells expressing the human FSH receptor in their membrane was assessed. For this scope, cells were stained and analyzed by FACS Aria Fusion instrument (BD Biosciences). Data analysis for staining experiments was performed using FlowJo software. The cell lines used for the staining were HEK 293-T FSHR Luciferase and the HEK-293-T WT without transfection. A primary FSHR conjugated antibody (Human FSH R Alexa Fluor®488-conjugated Antibody, R&D Systems, FAB65591G) was used for the staining.

The antibody was diluted in PBS + 2% FBS and incubated for 30 minutes at 4°C. After incubation, cells were washed with PBS + 2% FBS and resuspended in PBS + 2% FBS. Results were acquired by FACS instrument.

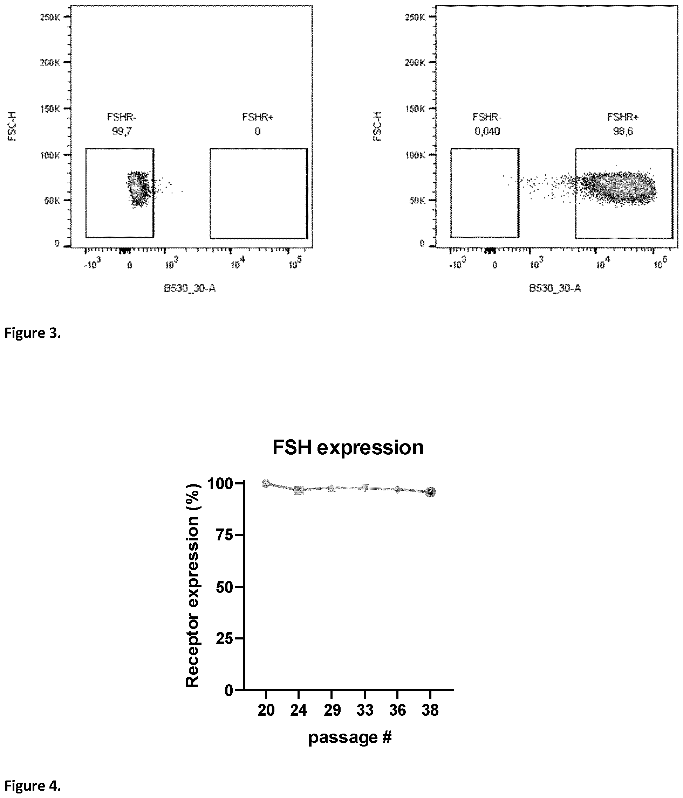

As shown in the figures 3, differences between non-transfected cells (HEK 293-T WT) and transfected cells (HEK 293-T LHCGR Luciferase) were visible. In fact, HEK 293-T FSHR Luciferase presented a 98.6% of cells positive for the FSH receptor versus the 0% positive stained cells in HEK 293-T WT sample. This staining experiment confirmed the success of the transfection. The stability of the FSH receptor expression over time was analyzed and, as shown in figure 4, resulted stable working window analyzed.

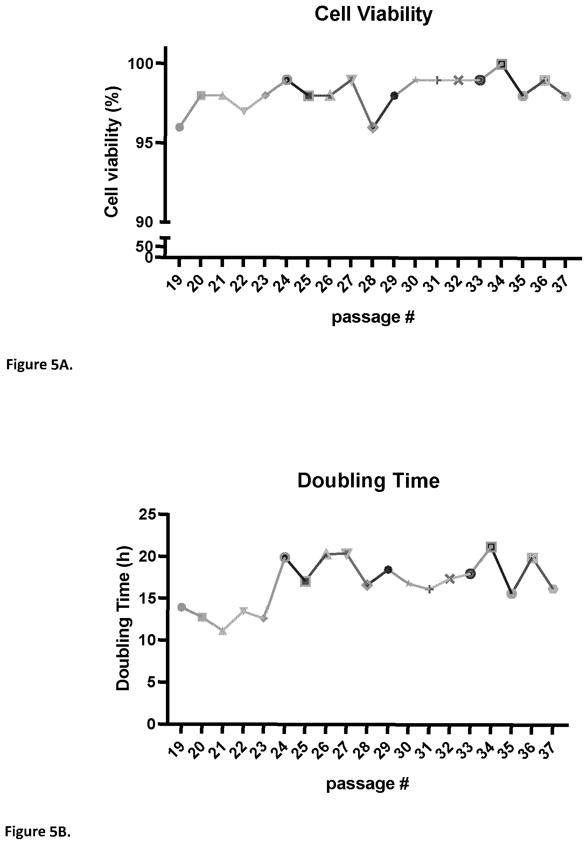

<u>HEK 293-T LHCGR Luciferase viability and doubling time.</u> Cell line viability and measurement was performed using Nucleocounter instrument (Chemometec). Results highlighted a viability >95% that remained stable during the range of passages analyzed (Figure 5A).

Doubling time (Figure 5B) was calculated using the formula:

$$\text{Doubling Time} = \frac{duration * \log(2)}{\log(Final\ Concentration) - \log(Initial\ Concentration)}$$

**Claims**

1. A mammalian cell for expressing a human FSH receptor and a luciferase reporter gene comprising:

   a. a human FSH receptor, comprising a nucleic acid molecule encoding the human FSH receptor under the control of a promoter for protein expression, and
   b. a reporter gene, comprising a nucleic acid molecule encoding for a luciferase reporter gene under the control of a cAMP Response Element (CRE).

2. The mammalian cell of claim 1, wherein the mammalian cell is a mammalian cell culture.

3. The mammalian cell of claim 2, wherein the mammalian cell culture is an adherent cell culture.

4. The mammalian cell of any one of claims 1-3, wherein the promotor for protein expression is a strong promoter for high level protein expression, such as CAG.

5. The mammalian cell of any one of claims 1-4, wherein:

   a. the nucleic acid molecule encoding the human FSH receptor is a vector comprising the FSH receptor gene and the gene for hygromycin resistance,
   b. the nucleic acid molecule encoding for the luciferase reporter gene is a vector comprising the luciferase reporter gene under the control of the cAMP Response Element (CRE) and
c. a gene for puromycin resistance.

6. The mammalian cell of any one of claims 1-5, wherein the mammalian cell is a human embryonic kidney (HEK) 293 cell.

7. The mammalian cell line of claim 6, wherein the HEK 293 cell is a HEK 293 T cell.

8. A method of producing a human FSH receptor and a luciferase reporter gene in a mammalian cell comprising:

   a. transfecting a mammalian cell with an isolated nucleic acid molecule encoding the FSH receptor under the control of a promoter for protein expression, wherein the mammalian cell line is a mammalian cell line stable transfected with a luciferase reporter gene under the control of a cAMP response element (CRE),
   b. adding a selectivity agent to the transfected cells of step a) to induce a stable expressing human FSH receptor cell line, and
   e. harvesting the cells stably expressing the human FSH receptor and the luciferase reporter.

9. The method of claim 8, wherein the selectivity agent is hygromycin.

10. The method of claim 8 or 9, wherein the promoter for expression of protein is a strong promoter for high level protein expression, such as CAG.

11. A method of determining the biological activity of r-hFSH samples, comprising

   a. contacting the sample with cell culture of the mammalian cell of claims 1-7,
   b. adding luminescence substrate for the luciferase enzyme to the cell culture,
   c. detecting the luminescence of the cells,
   d. quantifying the amount of luminescence, and
   e. determining the biological activity of r-hFSH samples.

| FSH ng/mL | FSH Luc2P/CRE | | | AVG | CV% |
|---|---|---|---|---|---|
| 2933,33 | 6802,03 | 6989,796 | 6649,616 | 6813,8 | 2,5 |
| 195,56 | 6623,377 | 6473,552 | 6838,046 | 6645,0 | 2,8 |
| 13,04 | 6726,804 | 7186,701 | 6792,929 | 6902,1 | 3,6 |
| 0,87 | 11162,79 | 10336,79 | 10755,67 | 10751,7 | 3,8 |
| 0,06 | 25081,08 | 24807,69 | 24130,44 | 24673,1 | 2,0 |

**Figure 1A.**

| FSH ng/mL | HEK-293T | | | AVG | %CV |
|---|---|---|---|---|---|
| 2933,33 | 26330,53 | 27485,88 | 25916,67 | 26577,7 | 3,1 |
| 195,56 | 26353,59 | 25124,65 | 25718,23 | 25732,2 | 2,4 |
| 13,04 | 25737,71 | 27410,47 | 26288,09 | 26478,8 | 3,2 |
| 0,87 | 26557,38 | 25424,66 | 26204,99 | 26062,3 | 2,2 |
| 0,06 | 26351,35 | 26260,16 | 26891,89 | 26501,1 | 1,3 |

**Figure 1B.**

**RGA FSH-Luc (816 ng/mL 1:12)**

**Figure 2.**

**Figure 3.**

**Figure 4.**

## Cell Viability

Figure 5A.

## Doubling Time

Figure 5B.

Europäisches
Patentamt

European
Patent Office

Office européen
des brevets

# EUROPEAN SEARCH REPORT

**Application Number**

EP 23 18 0633

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 1 466 620 A1 (AXXAM S R L [IT]) 13 October 2004 (2004-10-13) * abstract * * page 12, paragraph 94 * * page 18, paragraphs 134,135 * | 1-11 | INV. C07K14/72 C07K14/59 G01N33/76 C12Q1/6897 |
| X | US 2006/199806 A1 (FAILLI AMEDEO A [US] ET AL) 7 September 2006 (2006-09-07) * abstract * * page 32 – page 33; example 105 * | 1-11 | |
| X | PELLETIER ET AL: "Preparation of highly substituted @c-lactam follicle stimulating hormone receptor agonists", BIOORGANIC & MEDICINAL CHEMISTRY, ELSEVIER, AMSTERDAM, NL, vol. 13, no. 21, 1 November 2005 (2005-11-01), pages 5986-5995, XP005089327, ISSN: 0968-0896, DOI: 10.1016/J.BMC.2005.07.025 * abstract * * page 5988, right-hand column, last paragraph * | 1-11 | |
| X | NICOLE C. R. VAN STRATEN ET AL: "Identification of Substituted 6-Amino-4-phenyltetrahydroquinoline Derivatives: Potent Antagonists for the Follicle-Stimulating Hormone Receptor", JOURNAL OF MEDICINAL CHEMISTRY, vol. 48, no. 6, 1 March 2005 (2005-03-01), pages 1697-1700, XP055212214, ISSN: 0022-2623, DOI: 10.1021/jm0496761 * abstract * * page 1697, right-hand column, paragraph 2 * | 1-11 | |

**TECHNICAL FIELDS SEARCHED (IPC)**

C07K
G01N
C12Q

The present search report has been drawn up for all claims

| Place of search | Date of completion of the search | Examiner |
|---|---|---|
| Munich | 16 November 2023 | Grötzinger, Thilo |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

**EP 23 18 0633**

This annex lists the patent family members relating to the patent documents cited in the above-mentioned European search report.
The members are as contained in the European Patent Office EDP file on
The European Patent Office is in no way liable for these particulars which are merely given for the purpose of information.

**16-11-2023**

| Patent document cited in search report | Publication date | Patent family member(s) | Publication date |
|---|---|---|---|
| EP 1466620 A1 | 13-10-2004 | NONE | |
| US 2006199806 A1 | 07-09-2006 | NONE | |

EPO FORM P0459

For more details about this annex : see Official Journal of the European Patent Office, No. 12/82